# EUROPEAN PATENT SPECIFICATION

(11) **EP 1 488 757 B1**
(45) Date of publication and mention of the grant of the patent: **24.05.2006**
(21) Application number: 04253650.8
(22) Date of filing: 18.06.2004
(51) Int. Cl.: A61B 19/00

(54) **Cleaning test block**
Reinigungsprüfblock
Bloc pour tester un nettoyage

(30) Priority: 21.06.2003 GB 0314571
(43) Date of publication of application: 22.12.2004
(73) Proprietor: Trust Sterile Services Limited, Bellshill, Lanarkshire ML4 3NJ (GB)
(72) Inventor: Prior, Frank, Longniddry, East Lothian EH32 0QX (GB)
(74) Representative: McKechnie, Neil Henry

(56) References cited:
- WO-A-03/022336
- DE-U- 20 108 346
- US-A- 4 902 478
- US-A- 5 320 119

## Description

The present invention relates to apparatus that can be used to check the effectiveness of cleaning processes, in particular it can be used to check the effectiveness of instrumentation cleaning processes. The preferred embodiment has the instrumentation as a medical device or dental device which requires cleaning.

In the field of medicine, dentistry and scientific research, it is often necessary to use a wide range of instrumentation and, in most cases, this instrumentation needs to be cleaned and re-used regularly.

A number of instruments that are used in such cases have complicated configurations, such as narrow tubes, i.e., in the cases of endoscopes, etc., box joints in the cases of scissors, and coil shapes. Typically these are much more difficult to clean than objects where there are no hidden or difficult to reach surfaces.

Infections due to insufficient cleaning of these instruments are on the rise and have become a significant problem, particularly in relation to transmittable diseases, such as CJD. In response to this, there has been a great demand for the development of methods for preventing infections from incurring at the time of examination and treatment, and methods of cleaning instruments thoroughly. Many examples of methods for cleaning can be seen in the prior art, which use a wide range of substances and/or methodology. However, although there are number of methods suggested, there is no way of confirming that the methods are doing the job that is required of them. In particular, in real-life situations, it is important to know the every time instruments are put through a cleaning process, all of the contaminants are removed, and while a cleaning system may initially be very efficient, it is often the case that it becomes less efficient over time.

In certain cases, suggestions have been put forward to try and confirm that cleaning systems are working correctly. However, these typically consist of either testing for the presence of contaminants on the outer surfaces of an instrument after cleaning, or attaching something to the outer surface that can then be tested. Whilst this gives a good indication of whether the easily accessed surfaces have been cleaned sufficiently, it does not confirm that the more difficult to access areas, such as the inside of box joints or the inside of tubing, has also been cleaned or sterilised sufficiently.

DE 201 08 346 U1 discloses a device to test cleaning systems for tubular instruments.

It can be seen that it would be of great benefit to have a system or apparatus that allows the checking of cleaning systems, particularly the checking that cleaning systems are working appropriately in difficult to reach situations, such as insides of tubing and box joints.

It is a first object of the present invention to provide apparatus which can be used to check the effectiveness of instrumentation cleaning processes.

A further object of the present invention is to provide a method of testing a cleaning system to ensure that it is efficient.

According to a first aspect of the present invention, there is provided a test block according to claim 1.

Preferably the apparatus is made of stainless steel.

Optionally the surfaces of the test block may be coated in a material other than stainless steel.

Optionally at least one of the contoured surfaces represent the internal surfaces of a box joint.

Another option is that at least one of the contoured surfaces represent the inside of a tube.

Preferably the testing block is disc-shaped.

Preferably the sections of the test block are held together using screws and nuts, such that the screw is inserted into apertures which run through both sections, substantially perpendicularly to the contoured surfaces.

Alternatively, the sections can be held together using clamping means around the edges of the test block.

A further option is that the sections are held together magnetically.

According to a second aspect of the present invention, there is provided a method of testing a cleaning process, wherein:
- a test block which comprises two separable sections is taken through the cleaning process when the two sections are held together during the cleaning process, and wherein at least one of the two sections is covered with an amount of soiling means; and wherein the joint surfaces of the sections are contoured, so that when together they represent an internal surface of an instrument;
- the test block is then separated so that the contoured surfaces are exposed;
- the exposed contour surfaces are tested for the presence of contaminants.

Preferably the cleaning process involves placing the test block in one or more solutions.

Preferably standard biotechnological techniques are used to test for the presence of contaminants on the exposed contoured surfaces.

Most preferably the exposed contoured surfaces are tested for the presence of contaminants prior to being taken through the cleaning process, as well as post-cleaning.

Preferably the soiling means is pigs/porcine blood.

Optionally the soiling means is bovine blood or any other blood or blood-like substance (i.e., Edinburgh soil).

Optionally the soiling means is povidine iodine.

Please note that throughout this Application the term "cleaning process" relates to any process used for the decontamination of an instrument. This can be relating to the actual removal of contaminants from the instrument or, as in the case of sterilisation, the inactivation of certain contaminants.

In order to provide a better understanding of the present invention, we will now describe the invention by way of example only and with reference to the following Figures, in which:
Figure 1 shows a top view of the test block according to the present invention;
Figure 2 shows a top view of a contoured surface of one of the sections;
Figure 3 shows a section view through the side of a test block according to the present invention; and
Figure 4 shows a plan view of the layout of test blocks within a washer.

The inventor initially carried out a number of experiments to determine the effect of temperature in cleaning processes. Hot water at a range of temperatures between 55°C and 85°C was poured onto test plates for 5 seconds and an extraction carried out. It was found that any increase in temperature above 40°C has an adverse effect on protein removal. However, it was very difficult to determine what was happening in areas on instruments that were known to be difficult to clean.

The invention relates to a test block 1 which can be split into a lower section and an upper section 10. When the two sections 4 and 10 are brought together, part of the upper surface of the lower section 4 and the lower surface of the upper section 5 are brought into direct contact. However, the upper surface of the lower section 4 and the lower surface of the upper section 10 comprise contoured sections 3 which, when the lower section 4 and upper section 10 are brought together, reflect the internal or difficult to reach surfaces of certain instruments.

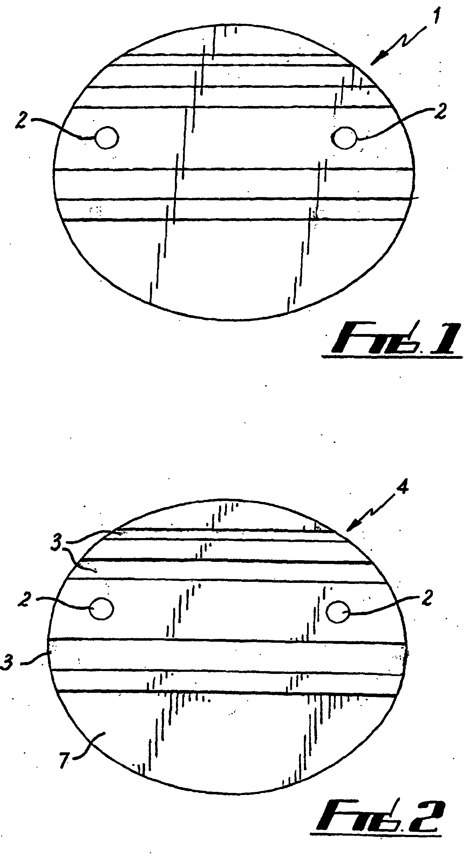

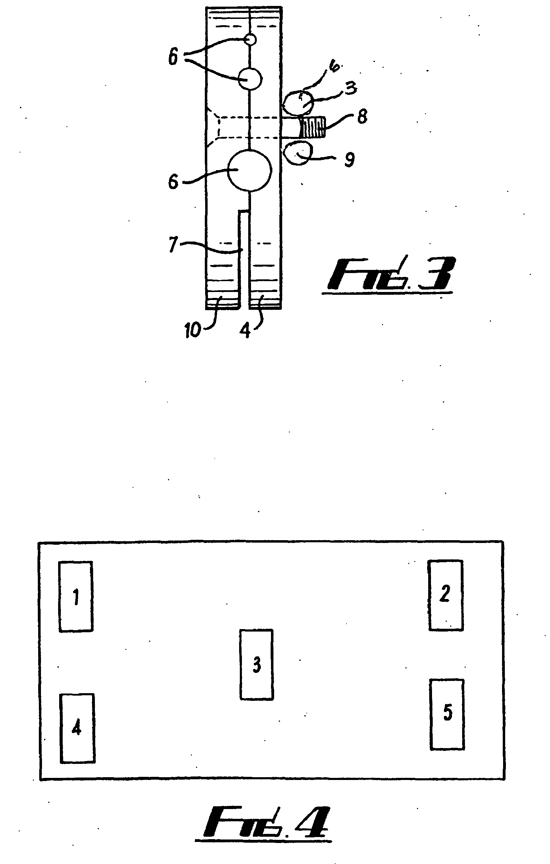

For example, the contoured sections can be produced to represent box joints in scissors, the typical internal diameters of catheter tubes, or a range of different surfaces. Typically this would be done by including a groove 7 to represent a box joint, where the groove 7 is 5mm in wide and 13mm in length. The diameter is typically set at 0.5mm, and the depth of the slot 5cm, although this can be varied. For catheter tubes, the combined contoured sections 3 result in an aperture 6 (as can be seen on Figure 3). In the preferred embodiment, a 0.2cm and 0.5cm half groove is drilled in each of the upper and lower sections 4, 10 which mimics the inside of a 0.2cm and 0.5cm catheter when the two halves are brought together.

As the majority of medical instruments are made from stainless steel, the preferred embodiment of the present invention has the test block 1 being made from stainless steel.

The test block 1 further comprises screw holes 2 which can be used to hold the upper section 10 and lower section 4 together. The screw 8 protrudes through the screw hole 2 and a nut 9 is tightened at one end of the screw 8. This means that upper section 10 and lower section 4 will not be parted if agitated, etc.

The test block 1 can be used to test cleaning processes to ensure that they are working correctly. Below we describe the typical steps in a cleaning test according to the present invention.

### Preliminary Separation

1. Spread 0.1ml of spiked pigs blood onto the lower section 4 of the stainless steel test block 1, covering all of the surface contours.
2. Allow the blood to air dry.
3. Drip 1ml of 50 to 100% alcohol onto the blood and allow to dry (preferably use 100% alcohol).
4. Affix the two sections 4 and 10 of the test block 1 together with the screw 8 and nut 9.
5. Stand in holders with box joint facing left and the grooves vertical.
6. Place the block into washer drier and run through the normal cleaning cycle, noting the position in which the test block has been placed.

### First Detection Test

1. After the block has been through the cleaning cycle, separate the lower section 4 and upper section 10 of the test block 1 by unscrewing the nut 9 from the screw 8 and removing the screw 8 from the screw hole 2.
2. Note any visible contamination remaining on the surfaces of the lower section 4, upper section 10 and record its position.
3. Test the surfaces of the lower section and upper section to determine whether any contamination is present.

### Testing for Contamination

There are various ways that we can test for contamination:
1. In the preferred embodiment the separated sections 4 and 10 are placed in an extraction container. An extraction solution is then added to the container and the container is rocked on a rocking bed at an agitation level of 7 for 30 minutes. Preferably the extraction solution is 50ml of 0.3% sodium carbonate (other extraction solutions can be used and aqueous solutions have been found to be the most effective. Preferably alkaline solutions are used which are approximately pH10 to 14 and ideally pH12).
2. The solution surrounding the test block 1 is taken, and transferred to a silica UV spectrometry cell.
3. The UV spectra is recorded over the range of 200 to 1,100nm.
4. The height of the peak (if present) is measured at 417nm.
5. The results are recorded.

In the preferred embodiment, only the section of the test block which had blood spread on it is used in the testing for contamination.

It can be seen that both the apparatus and the method described above provide significant benefits over the prior art. In particular, they allow the confirmation that cleaning processes are carrying out the cleaning to a level of satisfaction. In particular, it can be seen that the method allows for in process testing, such that different areas within a washer. In this case, 0.1ml of blood (preferably porcine, although other blood or blood-like solutions could be used) is spread on a number of test blocks 1 and allowed to dry. The blood is then sealed with 0.5ml of ethanol (50% to 100%) and allowed to dry. The blocks are then assembled and placed at predetermined positions within the tray of the washer (as in Figure 4).

It can also be seen that the present invention should not be considered as limiting, but merely as a basis for teaching one skilled in the art as to the various uses of the invention. In particular, there is no limitation on materials or the shapes of the test block. Also, the contours on the upper and lower sections of the test block can be varied to represent any necessary surface.

The present test can detect between 10⁻⁶ to 10⁻⁸ glml of protein. However, the level of detection can be increased by using a more sensitive method of protein detection, such as fluorimetry.

It can also be seen that the testing methods used to detect contamination can be of any type and the cleaning processes that the apparatus and testing is for can also be of any appropriate type.

## Claims

1. A test block (1) comprising a first section (10) and a second section (4), wherein the lower surface of the first section (10) is brought into contact with the upper surface of the second section (4), and wherein the respective upper and lower surfaces are contoured such that they represent one or more surfaces of an instrument, **characterized in that** at least one of the two sections is covered with an amount of soiling means.

2. A test block (1) as in Claim 1, which is made of stainless steel.

3. A test block (1) as in Claims 1 or 2, wherein the surfaces of the test block are coated in a material other than stainless steel.

4. A test block (1) as in any of the previous Claims, wherein at least one of the contoured surfaces (3) represents the internal surfaces of a box joint.

5. A test block (1) as in Claims 1 to 4, wherein at least one of the contoured surfaces (3) represents the inside of a tube.

6. A test block (1) as in any of the previous Claims, which is disc-shaped.

7. A test block (1) as in any of the previous Claims, wherein the sections of the test block are held together using screws and nuts, such that the screw (8) is inserted into apertures (2) which run through both sections (10, 4), substantially perpendicularly to the contoured surfaces (3).

8. A test block (1) as in Claims 1 to 6, wherein the sections (10, 4) can be held together using clamping means around the edges of the test block.

9. A test block (1) as in Claims 1 to 6, wherein the sections are held together magnetically.

10. A method of testing a cleaning process, wherein:
• a test block (1), which comprises two separable sections (10, 4), is taken through the cleaning process, wherein the two sections (10, 4) are held together during the cleaning process, and wherein at least one of the two sections is covered with an amount of soiling means; and wherein the joint surfaces of the sections are contoured, such that when together they represent an internal surface of an instrument;
• the test block (1) is then separated so that the contoured surfaces (3) are exposed;
• the exposed contoured surfaces (3) are then tested for the presence of contaminants.

11. A method as in Claim 10, wherein the cleaning process involves placing the test block (1) in one or more solutions.

12. A method as in Claims 10 or 11, wherein the exposed contoured surfaces (3) are tested for the presence of contaminants prior to being taken through the cleaning process, as well as post-cleaning.

13. A method as in Claims 10 to 12, wherein the soiling means is pigs blood.

14. A method as in Claims 10 to 12, wherein the soiling means is chosen from bovine blood or any other blood or blood-like substance.

15. A method as in Claims 10 to 12, wherein the soiling means is povidine iodine.

## Patentansprüche

1. Ein Prüfblock (1) bestehend aus einem ersten Abschnitt (10) und einem zweiten Abschnitt (4), wobei die unter Fläche des ersten Abschnitts (10) mit der oberen Fläche des zweiten Abschnitts (4) in Kontakt gebracht wird und wobei die Konturen der entsprechenden oberen und unteren Fläche so geformt sind, dass sie eine oder mehrere Oberflächen eines Instruments darstellen, charakterisiert **dadurch**, dass mindestens einer der beiden Abschnitte mit einer gewissen Menge Schmutzmittel versehen ist.

2. Ein Prüfblock (1) wie in Anspruch 1, der aus Edelstahl hergestellt ist.

3. Ein Prüfblock (1) wie in Anspruch 1 oder 2, wobei die Oberflächen des Prüfblocks mit einem. Material beschichtet sind, das nicht Edelstahl ist.

4. Ein Prüfblock (1) wie in einem der vorherigen Ansprüche, wobei mindestens eine der Konturenflächen (3) die Innenflächen einer Klemmdose darstellt.

5. Ein Prüfblock (1) wie in Anspruch 1 bis 4, wobei mindestens eine der Konturenflächen (3) das Innere eines Rohrs darstellt.

6. Ein Prüfblock (1) wie in einem der vorherigen Ansprüche, der scheibenförmig ist.

7. Ein Prüfblock (1) wie in einem der vorherigen Ansprüche, wobei die Abschnitte des Prüfblocks mittels Schrauben und Nuten zusammengehalten werden, so dass die Schraube (8) in die Öffnungen (2) eingeführt wird, die durch beide Abschnitte (10, 4) im Wesentlichen senkrecht zur Konturenfläche (3) verläuft.

8. Ein Prüfblock (1) wie in Anspruch 1-6, wobei die Abschnitte (10, 4) mittels Klemmmaterial um die Kanten des Prüfblocks zusammengehalten werden können.

9. Ein Prüfblock (1) wie in Anspruch 1 bis 6, wobei die Abschnitte magnetisch zusammengehalten werden.

10. Eine Methode zur Prüfung eines Reinigungsverfahrens, wobei:
• ein Prüfblock (1), der aus zwei trennbaren Abschnitten (10, 4) besteht, dem Reinigungsverfahren unterzogen wird, wobei die beiden Abschnitte (10, 4) während des Reinigungsverfahrens zusammengehalten werden und wobei mindestens einer der beiden Abschnitte mit einer gewissen Menge Schmutzmittel versehen ist und wobei die verbundenen Flächen der Abschnitte mit einer Kontur versehen sind, so dass wenn sie aneinander anliegen, eine Innenfläche eines Instruments darstellen.
• der Prüfblock (1) dann getrennt wird, so dass die Konturenflächen (3) freigelegt sind.
• die freigelegten Konturenflächen (3) dann auf das Vorhandensein von Verunreinigungssubstanzen geprüft werden.

11. Eine Methode wie in Anspruch 10, wobei das Reinigungsverfahren darin besteht, den Prüfblock (1) in eine oder mehrere Lösungen zu legen.

12. Eine Methode wie in Anspruch 10 oder 11, wobei die freigelegten Konturenflächen (3) auf das Vorhandensein von Verunreinigungssubstanzen geprüft werden, sowohl bevor sie dem Reinigungsverfahren unterzogen werden als auch nach der Reinigung.

13. Eine Methode wie in Anspruch 10 bis 12, wobei als Sehmutzmittel Schweineblut verwendet wird.

14. Eine Methode wie in Anspruch 10 bis 12, wobei als Schmutzmittel entweder Rinderblut, sonstiges Blut oder eine blutähnliche Substanz gewählt wird.

15. Eine Methode wie in Anspruch 10 bis 12, wobei als Schmutzmittel Povidon-Jod verwendet wird.

## Revendications

1. Bloc d'essai (1) comprenant une première section (10) et une deuxième section (4), dans lequel la surface inférieure de la première section (10) est amenée en contact avec la surface supérieure de la deuxième section (4), et dans lequel les surfaces supérieure et inférieure respectives sont profilées de sorte qu'elles représentent une ou plusieurs surfaces d'un instrument, **caractérisé en ce qu'**au moins une des deux sections est recouverte d'une quantité d'un moyen de souillure.

2. Bloc d'essai (1) suivant la revendication 1, lequel est réalisé en acier inoxydable.

3. Bloc d'essai (1) suivant la revendication 1 ou 2, dans lequel les surfaces du bloc d'essai sont revêtues d'un autre matériau que l'acier inoxydable.

4. Bloc d'essai (1) suivant l'une quelconque des revendications précédentes, dans lequel au moins une des surfaces profilées (3) représente les surfaces internes d'une charnière entrepassée.

5. Bloc d'essai (1) suivant l'une quelconque des revendications 1 à 4, dans lequel au moins une des surfaces profilées (3) représente l'intérieur d'un tube.

6. Bloc d'essai (1) suivant l'une quelconque des revendications précédentes, lequel a la forme d'un disque.

7. Bloc d'essai (1) suivant l'une quelconque des revendications précédentes, dans lequel les sections du bloc d'essai sont maintenues ensemble à l'aide de vis et d'écrous, de sorte que la vis (8) est introduite dans des ouvertures (2) qui passent à travers les deux sections (10. 4), pratiquement perpendiculairement aux surfaces profilées (3).

8. Bloc d'essai (1) suivant l'une quelconque des revendications 1 à 6, dans lequel les sections (10, 4) peuvent être maintenues ensemble à l'aide d'un moyen de serrage autour des bords du bloc d'essai.

9. Bloc d'essai (1) suivant l'une quelconque des revendications 1 à 6, dans lequel les sections sont maintenues ensemble par voie magnétique.

10. Procédé permettant d'essayer un processus de nettoyage, dans lequel :
• un bloc d'essai (1), qui comprend deux sections (10, 4) séparables, subit le processus de nettoyage, dans lequel les deux sections (10, 4) sont maintenues ensemble durant le processus de nettoyage, et dans lequel au moins une des deux sections est recouverte d'une quantité d'un moyen de souillure, et dans lequel les surfaces d'assemblage des sections sont profilées, de sorte que lorsqu'elles sont assemblées, elles représentent une surface interne d'un instrument ;
• le bloc d'essai (I) est ensuite séparé de sorte que les surfaces profilées (3) sont exposées, et
• les surfaces profilées exposées (3) sont ensuite essayées à la recherche de la présence de contaminants.

11. Procédé suivant la revendication 10, dans lequel le processus de nettoyage comprend le placement du bloc d'essai (1) dans une ou plusieurs solutions.

12. Procédé suivant la revendication 10 ou 11, dans lequel les surfaces profilées exposées (3) sont essayées à la recherche de la présence de contaminants avant de subir le processus de nettoyage, ainsi qu'un post-nettoyage.

13. Procédé suivant l'une quelconque des revendications 10 à 12, dans lequel le moyen de souillure est du sang de porc.

14. Procédé suivant l'une quelconque des revendications 10 à 12, dans lequel le moyen de souillure est choisi parmi du sang de bovidé ou tout autre sang ou toute autre substance similaire au sang.

15. Procédé suivant l'une quelconque des revendications 10 à 12, dans lequel le moyen de souillure est la polyvidone iodée.
